# EUROPEAN PATENT SPECIFICATION

(11) **EP 1 550 454 B1**
(45) Date of publication and mention of the grant of the patent: **09.03.2011**
(21) Application number: 04258069.6
(22) Date of filing: 22.12.2004
(51) Int. Cl.: A61K 38/19, A61K 39/395, A61K 41/00, A61P 37/06

(54) **Extracorporeal photopheresis in combination with anti-TNF treatment**
Extracorporeale Photopherese kombiniert mit anti-TNF Behandlung
Combinaison de la photophérèse extracorporéale et d'un traitement anti-TNF

(30) Priority: 23.12.2003 US 532076 P
(43) Date of publication of application: 06.07.2005
(73) Proprietor: THERAKOS, INC., Exton, PA 19341 (US)
(72) Inventor: Gregory, David, Bala Cynwyd, PA 19004 (US); Campbel, Kim, West Chester, PA 19382 (US); Giles-Komar, Jill, Dowingtown, PA 19335 (US); Harriman, Gregory, Paoli, PA 19301 (US)
(74) Representative: Mercer, Christopher Paul

(56) References cited:
- REDEI ISTVAN ET AL: "Salvage therapy with Infliximab for patients with severe acute and chronic GvHD" BLOOD, vol. 98, no. 11 Part 1, 16 November 2001 (2001-11-16), page 399a, XP009046456 & 43RD ANNUAL MEETING OF THE AMERICAN SOCIETY OF HEMATOLOGY, PART 1; ORLANDO, FLORIDA, USA; DECEMBER 07-11, 2001 ISSN: 0006-4971
- COURIEL D ET AL: "INFLIXIMAB FOR THE TREATMENT OF GRAFT-VERSUS-HOST DISEASE IN ALLOGENEIC TRANSPLANT RECIPIENTS: AN UPDATE" BLOOD, W.B.SAUNDERS COMPANY, ORLANDO, FL, US, vol. 96, no. 11, PART 1, 16 November 2000 (2000-11-16), page 400A,ABSTRNO1724, XP009028202 ISSN: 0006-4971
- AGARWAL R ET AL: "BIOTHERAPY: A NOVEL APPROACH IN THE TREATMENT OF PSORIASIS" DRUGS OF THE FUTURE, BARCELONA, ES, vol. 27, no. 11, 1 November 2002 (2002-11-01), pages 1071-1077, XP009036200 ISSN: 0377-8282
- LEBWOHL MARK: "Combining the new biologic agents with our current psoriasis armamentarium." JOURNAL OF THE AMERICAN ACADEMY OF DERMATOLOGY. AUG 2003, vol. 49, no. 2 Suppl, August 2003 (2003-08), pages S118-S124, XP002325072 ISSN: 0190-9622
- "Combination therapy with PUVA and etanercept in the treatment of severe psoriasis" JOURNAL OF THE AMERICAN ACADEMY OF DERMATOLOGY, C.V. MOSBY, ST. LOUIS, MO, US, vol. 52, no. 3, March 2005 (2005-03), page P179, XP004788755 ISSN: 0190-9622
- ROOK A H; ET AL: "PHOTOPHERESIS: CLINICAL APPLICATIONS AND MECHANISM OF ACTION" JOURNAL OF INVESTIGATIVE DERMATOLOGY SYMPOSIUM PROCEEDINGS, vol. 4, no. 1, September 1999 (1999-09), pages 85-90, XP008005233 CAMBRIDGE, US

## Description

### BACKGROUND

The present application relates to treatment of immune-related disorders.

Autoimmune diseases involve inappropriate activation of immune cells that are reactive against self tissue. These activated immune cells promote the production of cytokines and autoantibodies involved in the pathology of the diseases. Other diseases involving T-cells include Graft versus Host Disease (GVHD) which occurs in the context of transplantation. In GVHD donor T-cells reject recipient's tissues and organs by mounting an attack against the recipient's body. A host of other diseases involve disregulation of the host immune system. Some are best treated with pharmaceuticals, some with biologicals, others with treatments such as extracorporeal photophoresis, and yet others have very limited treatment options.

Extracorporeal photopheresis (ECP) has been shown to be an effective therapy in certain T-cell mediated diseases. In the case of GVHD, photopheresis has been used as a treatment in association with topical triamcinolone oinment, antifungal, antiviral, antibiotics, immuneglobulins, and methotrexate. ECP has also been used with immunosuppressive agents such as mycophenolate mofetil, tacrolimus, prednisone, cyclosporine, hydroxychloroquine, steroids, FK-506, and thalidomide for cGVHD and refractory cGVHD. For solid organ transplants, ECP has been used in conjunction with immunosuppressive agents to reduce the number of acute allograft rejection episodes associated with renal allografts and cardiac transplants. For example, ECP has been used with OKT3 and/or the immunosuppressive agents prednisone, azathioprine, and cyclosporine to reverse acute renal allograft rejection. ECP has also been used with cyclophosphamide, fractionated total body irradiation, and etoposide for allogeneic marrow transplantation for acute myeloid leukemia, acute lymphoblastic leukemia, chronic myeloid leukemia, non-Hodgkin's lymphoma, or severe aplastic anemia.

Despite current combination use of ECP with other therapeutic agents, there remains a need for a combination of ECP with a concomitant agent to treat patients having immune-mediated diseases, atopic hypersensitivities or GVHD, where existing treatments are not as effective as they otherwise might be or may have serious side effects or are difficult to administer at the levels in which either treatment by itself is delivered.

Monocytes and macrophages secrete tumor necrosis factor (TNF-α) as a cytokine in response to endotoxin or other stimuli. TNF-α is a soluble homotrimer of 17 kD protein subunits. Cells other than monocytes or macrophages also make TNF-α. For example, human non-monocytic tumor cell lines produce TNF. TNF-α has been implicated in inflammatory diseases, autoimmune diseases, viral, bacterial and parasitic infections, malignancies, and/or neurogenerative diseases and is a useful target for specific biological therapy in diseases, such as rheumatoid arthritis and Crohn's disease. The administration of antibodies as a treatment has not been problem free. For example, using a TNF-α-antagonist has, in some cases, contributed to the occurrence of serious infections. Reducing the dosage of such substances would reduce complications of the treatment.

Successful use of TNF antagonists such as infliximab and etanercept in combination with methotrexate (MTX) for arthritis treatment has been reported and several of these agents are currently approved by regulatory agencies for this use. While these agents have been a large step forward for the treatment of arthrititis, for a variety of reasons there is a substantial minority of patients who either do not respond or respond weakly to these agents. Difficult treatment issues still remain for patients with rheumatoid arthritis. Many current treatments have a high incidence of side effects or cannot completely prevent disease progression. Even though agents such as methotrexate, steroids and other chemotherapeutic agents have a long history of use in the treatment of various immunologic diseases, including rheumatoid arthritis, patients using these compounds can have major toxic effects, such as hepatic, pulmonary, renal and bone marrow abnormalities. Patients using these compounds may also have minor side effects such as stomatitis, malaise, nausea, diarrhea, headaches and mild alopecia; however, these can be treated with folate supplementation. Thus, there is a need for safer combination treatment for arthritis with TNF antagonist besides the currently approved products.

Redei et al., "Salvage therapy with infliximab for patients with severe acute and chronic GVHD", Blood, vol. 98, no. 11. Part 1, page 399a, disclose administration of infliximab at a dose of 10 mg/kg_and administration of (PUVA) (psolaren + UVA treatment - an *"in vitro* treatment).

Couriel et al., "Infliximab for the treatment of graft-versus-host disease in allogeneic transplant recipients: an update", Blood, vol. 96, no. 11. Part 1, page 400A, disclose administration of Infliximab at 10 mg/kg and additional salvage therapy including photopheresis.

Rook et al., "Photopheresis: Clinical applications and mechanism of action" Journal of Investigative Dermatology Symposium Proceedings, vol. 4, no. 1, pages 85-90 summarise the use of photopheresis to treat cutaneous T cell lymphoma, allograft rejection and autoimmune disease.

### SUMMARY OF THE INVENTION

The present invention provides a combined product comprising:
a) an *ex vivo* population of cells that has been subjected to an apoptosis-inducing treatment *ex vivo*; and
b) infliximab in a dose that does not exceed 3mg/kg,
for combined, simultaneous or sequential administration to a patient for treating an autoimmune disease or ameliorating one or more symptoms thereof.

In another aspect, the present invention provides a composition comprising infliximab for use in a method of treating a patient with an autoimmune disorder or the predisposition for an autoimmune disorder wherein said method comprises testing the patient to determine whether the patient has an autoimmune disorder, and administering infliximab in a dose that does not exceed 3mg/kg and ECP if such patient has an autoimmune disorder or a predisposition to such disorder.

The invention provides a product for improved treatment for GVHD and other immune related disorders by enabling lower doses of TNFα inhibitor (and thus lessening toxicity), elongating time between infusions, and increasing the efficacy of both the cellular treatment (e.g., ECP) and the TNFα inhibitor.

### DETAILED DESCRIPTION OF THE INVENTION

The terms "subject" or "patient" are used interchangeably and refer to an animal, preferably a mammal and more preferably a human.

A "cell population" generally includes a cell type found in blood. The term may include one or more types of blood cells, specifically, red blood cells, platelets, and white blood cells. A cell population may comprise subtypes of white blood cells, for example, T-cells, dendritic cells, B-cells, etc. In one embodiment, a cell population may comprise a mixture or pool of cell types. Alternatively, a cell population may comprise a substantially purified type of cells, for example, T-cells or dendritic cells.

"ECP procedure" or "ECP" refers to extracorporeal photopheresis, also known as extracorporeal phototherapy. It is a treatment of a population of cells that has been subjected to UVA light and a photoactivatable compound. Preferably the population of cells is from an organ or tissue; more preferably, the population of cells is a portion of blood; and most preferably, the population of cells is a buffy coat. ECP is sometimes used to refer to a process in which a cell population has been subjected to an apoptosis-inducing procedure with UVA light in the presence of a DNA cross linking agent such as a psoralen (preferably, 8-MOP).

The side effects that are referred to in this specification are the unwanted and adverse effects of a therapeutic concomitant agent. Adverse effects are always unwanted, but unwanted effects are not necessarily adverse. An adverse effect from a therapeutic agent might be harmful or uncomfortable or risky. Side effects from administration of anti-TNF-α treatments may include, but are not limited to, risk of infection and hypersensitivity reactions. Other side effects range from nonspecific symptoms such as fever or chills, pruritus or urticaria, and cardiopulmonary reactions such as chest pain, hypotension, hytertension or dyspnea, to effects such as myalgia and/or arthralgia, rash, facial, hand or lip edema, dysphagia, sore throat, and headache. Yet other side effects can include, but are not limited to, abdominal hernia, splenic infarction, splenomegaly, dizziness, upper motor neuron lesions, lupus erythematosus syndrome, rheumatoid nodules, ceruminosis, abdominal pain, diarrhea, gastric ulcers, intestinal obstruction, intestinal perforation, intestinal stenosis, nausea, pancreatitis, vomiting, back pain, bone fracture, tendon disorder or injury, cardiac failure, myocardial ischema, lymphoma, thrombocytopenia, cellulitis, anxiety, confusion, delirium, depression, somnolence, suicide attempts, anemia, abscess, bacterial infections, and sepsis.

The terms "disorder" and "disease" are used interchangeably in this specification. The term "atopic disease" is used interchangeably with the term "inflammatory disorder" to refer to a condition in a subject characterized by inflammation such as chronic inflammation. Autoimmune disorders may or may not be associated with inflammation. Moreover, inflammation may or may not be caused by an autoimmune disorder. Thus, certain disorders may be characterized as both autoimmune and inflammatory disorders.

The concomitant agents of this invention include an immunomodulatory agent relating to TNF-α. The immunomodulatory agent used in the combined products of the invention is a TNF-α antagonist which is REMICADE ®_(infliximab).

REMICADE ® (infliximab), decreases, blocks, inhibits, abrogates or interferes with TNF-α activity *in vitro, in situ* and/or preferably *in vivo*.

The TNF-α antagonist of the invention is preferably administered by parenteral, subcutaneous, intramuscular, intravenous, or intraarticular means. Other means are also possible including intrabronchial, intraabdominal, intracapsular, intracartilaginous, intracavitary, intracelial, intracerebellar, intracerebroventricular, intracolic, intracervical, intragastric, intrahepatic, intramyocardial, intraosteal, intrapelvic, intrapericardiac, intraperitoneal, intrapleural, intraprostatic, intrapulmonary, intrarectal, intrarenal, intraretinal, intraspinal, intrasynovial, intrathoracic, intrauterine, intravesical, bolus, vaginal, rectal, buccal, sublingual, intranasal, or transdermal means.

In an embodiment of the invention that provides compositions for use in combination therapies for prevention, treatment or amelioration of one or more symptoms associated with an autoimmune disease in a subject, the therapies include administering to a subject a population of cells that has been subjected to an apoptosis inducing treatment, for example, a population of cells that has been subjected to extracorporeal photopheresis (ECP), and a TNFα antagonist.

The combination of ECP and a concomitant agent (i.e., TNFα antagonist) produces a better therapeutic effect in a subject than either treatment alone. In certain embodiments, the combination of ECP and a concomitant agent achieves a 2 fold or more (and preferably a 10 to 20 fold) better therapeutic effect in a subject with an autoimmune disease, disease, GVHD, or transplant rejection than either treatment alone. In other embodiments, the combination of ECP and a TNF antagonists has a more than additive effect in a subject with an autoimmune disease, GVHD and implant or transplant rejection. The combination products for use in therapies of the invention enable less frequent administration of ECP to a subject with an autoimmune to achieve a therapeutic effect; enable lower dosages of the TNF-α antagonist utilized in conjunction with ECP for the prevention or treatment of an autoimmune immune disease, atopic disease, or GVHD and/or less frequent administration of such TNF-α antagonist to a subject with an autoimmune disease, or GVHD to achieve a therapeutic effect. They reduce or avoid unwanted or adverse side effects associated with the administration of current single agent therapies and/or existing combination therapies for autoimmune disease, or GVHD, which in turn improves patient compliance with the treatment protocol.

Lowering the dosages and/or frequency of administration of ECP or concomitant agent to a subject with an autoimmune disease improves the quality of life of a patient undergoing such therapy. The dosages and/or frequency of administration of ECP or concomitant agent to a subject with an autoimmune or disease can be lowered and still achieve a 20% or more (and preferably 90% - 98% or greater reduction) in the inflammation of a particular organ, tissue or joint in the patient.

In one embodiment, the ECP is used in combination with monoclonal anti- TNF-α antibodies. The most preferred monoclonal anti-TNF antibody is infliximab (REMICADE®). The TNF-α antagonist used in the compositions of the invention is infliximab (REMICADE ®). Infliximab (REMICADE®)) is a chimeric monoclonal antibody that binds to tumor necrosis factor alpha (TNF-α).

In another preferred embodiment of the invention, REMICADE ® (infliximab) is supplied as a sterile and lyophilized powder for intravenous infusion to be reconstituted with 10 ml sterile water for injection. Each single-use vial of REMICADE® (infliximab) contains 100 mg infliximab, 500 mg sucrose, 0.5 mg polysorbate 80, 2.2 mg monobasic sodium phosphate and 6.1 mg dibasic sodium phosphate. According to The Physician's Desk Reference (55ed., 2001), the total dose of the reconstituted product must be further diluted to 250 ml with 0.9% Sodium Chloride Injection, USP, with the infusion concentration ranging between 0.4 mg/ml and 4 mg/ml. In the invention, the dose does not exceed 3 mg/kg. In certain preferred embodiments, REMICADE® (infliximab) is for administration by intravenous infusion followed with an additional dose at 2 and 6 weeks after the first infusion then every 8 weeks thereafter.

In an embodiment of the invention, REMICADE® (infliximab) is administered at a dose of about 2.5 mg/kg in combination with ECP. In preferred embodiments the amount of REMICADE is significantly lower in order to lower toxicity when the synergy is strong and the disease warrants (such as GVHD). In these embodiments the frequency of ECP and or antiTNF α treatment is reduced by 20%, more preferably 40%, and most preferably by at least 50%.

ECP is sequentially administered, in either order, with the TNF α antagonist(s) of this invention. This may also be done cyclically. Cyclical therapy involves the administration of a concomitant agent for a period of time, followed by the administration of a cell population comprising apoptotic cells for a period of time and repeating this sequential administration. Preferably, a cell population comprising apoptotic cells (such as one obtains during ECP) is administered at least about 15 - 60 minutes before or after a concomitant agent. The cell population comprising apoptotic cells may, however, be administered at much greater intervals before or after a TNF α antagonist. For example, in some cases it is possible to administer the cell population comprising apoptotic cells at least about 1 day to 30 days or more before or after the administration of a concomitant agent and still obtain the beneficial effect of the combination therapy.

The cell populations useful in the therapy of the methods disclosed herein comprise "apoptotic cells," which include cells and cell bodies, i.e., apoptotic bodies, that exhibit, or will exhibit, one or more apoptosis-characterizing features. An apoptotic cell may comprise any cell that is in the Induction phase, Effector phase, or the Degradation phase. The cell populations disclosed herein may also comprise cells that have been treated with an apoptotis-inducing agent that are still viable. Such cells may exhibit apoptosis-characterizing features at some point, for example, after administration to the subject.

ECP directly induces significant levels of apoptosis. This has been observed, for example, in lymphocytes of CTCL, GVHD, and scleredema patients. The apoptotic cells contribute to the observed clinical effect.

Apoptosis-characterizing features may include, but are not limited to, surface exposure of phosphatidylserine, as detected by standard, accepted methods of detection such as Annexin V staining; alterations in mitochondrial membrane permeability measured by standard, accepted methods (e.g., Salvioli et al., 411 FEBS LETTERS 77-82 (1997)); evidence of DNA fragmentation such as the appearance of DNA laddering on agarose gel electrophoresis following extraction of DNA from the cells (Teiger et al., 97 J. CLIN. INVEST. 2891-97 (1996)), or by in situ labeling (Gavrieli et al., 1992, referenced above).

The cell population for use in the present invention are induced to become apoptotic *ex vivo,* i.e., extracorporeally, and are compatible with those of the subject, donor, or recipient. A cell population may be prepared from substantially any type of mammalian cell including cultured cell lines. For example, a cell population may be prepared from a cell type derived from the mammalian subject's own body or from an established cell line. Specifically, a cell population may be prepared from white blood cells of blood compatible with that of the mammalian subject, more specifically, from the subject's own white blood cell and even more specifically, from the subject's own T-cells.

A cell population may also be prepared from an established cell line. A cell line that may be useful in the methods of the present invention includes, for example, Jurkat cells (ATCC No. TIB-152). Other cells lines appropriate for use in accordance with the present invention may be identified and/or determined by those of ordinary skill in the art. The cell population may be prepared extracorporeally prior to administration to the subject, donor, or recipient. Thus, in one embodiment, an aliquot of the subject's blood, recipient's blood, or the donor's blood may be withdrawn, e.g. by venipuncture, and at least a portion of the white cells thereof subjected extracorporeally to apoptosis-inducing conditions.

In one embodiment, the cell population may comprise a particular subset of cells including, but not limited to dendritic cells, CD25⁺ CD4 T-regulatory cells, and CD4⁺ T-cells. The separation and purification of blood components is well known to those of ordinary skill in the art. Indeed, the advent of blood component therapy has given rise to numerous systems designed for the collection of specific blood components. Several of these collection systems are commercially available from, for example, Immunicon Corp. (Huntingdon Valley, PA), Baxter International (Deerfield, IL), and Dynal Biotech (Oslo, Norway).

Immunicon's separation system separates blood components using magnetic nanoparticles (ferrofluids) coated with antibodies that conjugate, i.e., form a complex, to the target components in a blood sample. The blood sample is then incubated in a strong magnetic field and the target complex migrates away from the rest of the sample where it can then be collected. *See, e.g.*, U.S. Patent Nos. 6,365,362; 6,361,749; 6,228,624; 6,136,182; 6,120,856; 6,013,532; 6,013,188; 5,993,665; 5,985,153; 5,876,593; 5,795,470; 5,741,714; 5,698,271; 5,660,990; 5,646,001; 5,622,831; 5,597,531; 5,541,072; 5,512,332; 5,466,574; 5,200,084; 5,186,827; 5,108,933; and 4,795,698.

Dynal's Dynabeads® Biomagnetic separation system separates blood components using magnetic beads coated with antibodies that conjugate to the target components in a blood sample, forming a Dynabeads-target complex. The complex is then removed from the sample using a Magnetic Particle Concentrator (Dynal MPC®). Several different cell types may be collected using this separation system, including for example, dendritic cells derived from monocytes (Monocyte Negative Isolation Kit, Prod. No. 113.09), dendritic cells derived from CD34⁺ cells (Dynal® CD34 Progenitor Cell Selection System, Prod. No. 113.01), and human monocytes (Dynabeads® CD 14: Monocyte Positive Isolation for Molecular Analysis, Prod. Nos. 111.11 or 111.12). T cells and T cell subsets can also be positively or negatively isolated or depleted from whole blood, buffy coat, gradient mononuclear cells or tissue digests using, for example, CELLection™ CD2 Kit (Prod.No 116.03), Dynabeads® M-450 CD2 (Prod. No 111.01/02), Dynabeads® CD3 (Prod.No 111.13/14), Dynabeads® plus DETACHaBEAD (Prod. No. 113.03), Dynabeads® M-450 CD4 (Prod.No 111.05/06), CD4 Negative Isolation Kit (T helper/inducer cells) (Prod. No. 113.17), CD8 Positive Isolation Kit (Prod. No. 113.05), Dynabeads® CD8 (Prod. No. 111.07/08), CD8 Negative Isolation Kit (Prod. No. 113.19), T Cell Negative Isolation Kit (Prod. No. 113.11), Dynaheads® CD25 (Prod. No 111.33/34), and Dynabeads® CD3/CD28 T Cell Expander (Prod. No. 111.31). Baxter International has developed several apheresis systems based on the properties of centrifugation, including the *CS-3000* blood cell separator, the *Amicus* separator, and the *Autopheresis-C* system. The *CS-3000* Plus blood cell separator collects both cellular apheresis products and plasma. It comprises a continuous-flow separator with a dual-chamber centrifugal system that collects apheresis products. The *Amicus* operates in either a continuous-flow or intermittant-flow format to collect single donor platelets and plasma. The *Autopheresis-C* system is designed for the collection of plasma from donors and can collect more than 250 mL of plasma. *See generally*, U.S. Patent Nos. 6,451,203; 6,442,397; 6,315,707; 6,284,142; 6,251,284; 6,033,561; 6,027,441; and 5,494,578.

In the most preferred embodiment of the invention, ECP is used to induce apoptosis. This involves a photoactivatable compound added to a cell population *ex vivo.* The photosensitive compound may be administered to a cell population comprising blood cells following its withdrawal from the subject, recipient, or donor, as the case may be, and prior to or contemporaneously with exposure to ultraviolet light. The photosensitive compound may be administered to a cell population comprising whole blood or a fraction thereof provided that the target blood cells or blood components receive the photosensitive compound. In another embodiment, a portion of the subject's blood, recipient's blood, or the donor's blood could first be processed using known methods to substantially remove the erythrocytes and the photoactive compound may then be administered to the resulting cell population comprising the enriched leukocyte fraction.

In an alternative to the present invention, the photoactivatable compound may be administered *in vivo.* The photosensitive compound, when administered to a cell population comprising the subject's blood, recipient's blood, or the donor's blood, as the case may be, *in vivo* may be administered orally, but also may be administered intravenously and/or by other conventional administration routes. The oral dosage of the photosensitive compound may be in the range of about 0.3 to about 0.7 mg/kg., more specifically, about 0.6 mg/kg. When administered orally, the photosensitive compound may be administered at least about one hour prior to the photopheresis treatment and no more than about three hours prior to the photopheresis treatment.

Photoactivatable compounds for use in accordance with the present invention include, but are not limited to, compounds known as psoralens (or furocoumarins) as well as psoralen derivatives such as those described in, for example, U.S. Pat. No. 4,321,919 and U.S. Pat. No. 5,399,719. Preferred compounds include 8-methoxypsoralen; 4,5'8-trimethylpsoralen; 5-methoxypsoralen; 4-methylpsoralen; 4,4-dimethylpsoralen; 4-5'-dimethylpsoralen; 4'-aminomethyl-4,5',8-trimethylpsoralen; 4'-hydroxymethyl-4,5',8-trimethylpsoralen; 4',8-methoxypsoralen; and a 4'-(omega-amino-2-oxa) alkyl-4,5',8-trimethylpsoralen, including but not limited to 4'-(4-amino-2-oxa)butyl-4,5',8-trimethylpsoralen. In one embodiment, the photosensitive compound that may be used comprises the psoralen derivative, amotosalen (S-59) (Cerus, Corp., Concord, CA). In another embodiment, the photosensitive compound comprises 8-methoxypsoralen (8 MOP).

The cell population to which the photoactivatable compound has been added is treated with a light of a wavelength that activates the photoactivatable compound. The treatment step that activates the photoactivatable compound is preferably carried out using long wavelength ultraviolet light (UVA), for example, at a wavelength within the range of 320 to 400 nm. The exposure to ultraviolet light during the photopheresis treatment preferably is administered for a sufficient length of time to deliver about 1-2 J/cm² to the cell population.

Extracorporeal photopheresis apparatus useful in the invention include those manufactured by Therakos, Inc., (Exton, PA) under the name UVAR®. A description of such an apparatus is found in U.S. Pat. No. 4,683,889. The UVAR® System uses a treatment system and consists of three phases including: 1) the collection of a buffy-coat fraction (leukocyte-enriched), 2) irradiation of the collected buffy coat fraction, and 3) reinfusion of the treated white blood cells. The collection phase has six cycles of blood withdrawal, centrifugation, and reinfusion steps. During each cycle, whole blood is centrifuged and separated in a pheresis bowl. From this separation, plasma (volume in each cycle is determined by the UVAR®. Instrument operator) and 40 ml buffy coat are saved in each collection cycle. The red cells and all additional plasma are reinfused to the patient before beginning the next collection cycle. Finally, a total of 240 ml of buffy coat and 300 ml of plasma are separated and saved for UVA irradiation.

The irradiation of the leukocyte-enriched blood within the irradiation circuit begins during the buffy coat collection of the first collection cycle. The collected plasma and buffy coat are mixed with 200 ml of heparinized normal saline and 200 mg of UVADEX® (water soluble 8-methoxypsoralin). This mixture flows in a 1.4 mm thick layer through the PHOTOCEPTOR® Photoactivation Chamber, which is inserted between two banks of UVA lamps of the PHOTOSETTE®. PHOTOSETTE® UVA lamps irradiate both sides of this UVA-transparent PHOTOCEPTOR® chamber, permitting a 180-minute exposure to ultraviolet A light, yielding an average exposure per lymphocyte of 1-2 J/cm². The final buffy coat preparation contains an estimated 20% to 25% of the total peripheral blood mononuclear cell component and has a hematocrit from 2.5% to 7%. Following the photoactivation period, the volume is reinfused to the patient over a 30 to 45 minute period. U.S. Patent Application No. 09/480,893 describes another system for use in ECP administration. U.S. Patent Nos. 5,951,509; 5,985,914; 5,984,887, 4,464,166; 4,428,744; 4,398,906; 4,321,919; PCT Publication Nos. WO 97/36634; and WO 97/36581 also contain description of devices and methods useful in this regard.

Another system that may be useful in using the present invention is described in WO01/80924. That system includes an apparatus by which the net fluid volume collected or removed from a subject may be reduced during ECP. The effective amount of light energy that is delivered to a cell population may be determined using the methods and systems described in U.S. Patent No. 6,219,584.

A variety of other methods for inducing apoptosis in a cell population are well-known and may be adopted for use in the present invention. One such treatment comprises subjecting a cell population to ionizing radiation (gamma-rays, x-rays, etc.) and/or non-ionizing electromagnetic radiation including ultraviolet light, heating, cooling, serum deprivation, growth factor deprivation, acidifying, diluting, alkalizing, ionic strength change, serum deprivation, irradiating, or a combination thereof. Alternatively, apoptosis may be induced by subjecting a cell population to ultrasound.

Yet another method of inducing apoptosis comprises the extracorporeal application of oxidative stress to a cell population. This may be achieved by treating the cell population, in suspension, with chemical oxidizing agents such as hydrogen peroxide, other peroxides and hydroperoxides, ozone, permanganates, periodates, and the like. Biologically acceptable oxidizing agents may be used to reduce potential problems associated with residues and contaminations of the apoptosis-induced cell population so formed.

In preparing the apoptosis-induced cell population, care should be taken not to apply excessive levels of oxidative stress, radiation, drug treatment, etc., because otherwise there may be a significant risk of causing necrosis of at least some of the cells under treatment. Necrosis causes cell membrane rupture and the release of cellular contents often with biologically harmful results, particularly inflammatory events, so that the presence of necrotic cells and their components along with the cell population comprising apoptotic cells is best avoided. Appropriate levels of treatment of the cell population to induce apoptosis, and the type of treatment chosen to induce apoptosis are readily determinable by those skilled in the art.

One process according to the present invention involves the culture of cells from the subject, or a compatible mammalian cell line. The cultured cells may then be treated extracorporeally to induce apoptosis and to create a cell population therein. The extracorporeal treatment may be selected from the group consisting of antibodies, chemotherapeutic agents, radiation, extracorporeal photopheresis, ultrasound, proteins, and oxidizing agents. The cells, suspended in the subject's plasma or another suitable suspension medium, such as saline or a balanced mammalian cell culture medium, may then be administered as indicated below.

Methods for the detection and quantitation of apoptosis are useful for determining the presence and level of apoptosis in the preparation to be administered to the subject. The number of apoptotic cells in a cell population required to obtain the required clinical benefit in a subject may vary depending on the source of cells, the subject's condition, the age and weight of the subject and other relevant factors, which are readily determinable by well-known methods. Preferably, the number of apoptotic cells that are administered to a patient are .1 to 50 billion, more preferably 1 to 10, and most preferably 2.5 to 7.5 billion.

In one embodiment, cells undergoing apoptosis may be identified by a characteristic 'laddering' of DNA seen on agarose gel electrophoresis, resulting from cleavage of DNA into a series of fragments. In another embodiment, the surface expression of phosphatidylserine on cells may be used to identify and/or quantify an apoptosis-induced cell population. Measurement of changes in mitochondrial membrane potential, reflecting changes in mitochondrial membrane permeability, is another recognized method of identification of a cell population. A number of other methods of identification of cells undergoing apoptosis and of a cell population, many using monoclonal antibodies against specific markers for a cell population, have also been described in the scientific literature.

The administration of apoptotic cells of the present invention and TNF-α antagonist finds utility in treating arthritis and other autoimmune diseases. They are also useful in the treatment or prophylaxis of at least one autoimmune-related disease in a cell, tissue, organ, animal, or patient including, but not limited to, acute and chronic immune and autoimmune pathologies, such as systemic lupus erythematosus, rheumatoid arthritis, thyroidosis, graft versus host disease, scleroderma, diabetes mellitus, Graves' disease, and the like; and atopic diseases, including chronic inflammatory pathologies such as sarcoidosis, chronic inflammatory bowel disease, ulcerative colitis, and Crohn's pathology.

By way of example, solid organ transplantion is more benefically treated by this invention than by administration of a TNF-α antagonist alone. Acute solid organ transplantion rejection occurs in 30% to 60% of patients after lung transplantation and to a lower degree with liver, kidney, heart etc. due to the success of immunosuppressive agents. The lymphocyte (cell)-mediated immune reaction against transplantation antigens , is the principal mechanism of acute rejection. A delayed or chronic rejection causes graft destruction in months to years after transplantation and is characterized by vascular destruction leading to necrosis of the transplanted tissue. This rejection is not currently suppressed to any large degree by standard regimens and thus the need for more sustainable immune tolerance is a significant unmet need.

Late graft deterioration occurs occasionally, and this chronic type of rejection often progresses insidiously despite increased immunosuppressive therapy. The pathologic picture differs from that of acute rejection. The arterial endothelium is primarily involved, with extensive proliferation that may gradually occlude the vessel lumen, resulting in ischemia and fibrosis of the graft.

Immunosuppressants are currently widely used to control the rejection reaction and are primarily responsible for the success of transplantation. However, these drugs suppress all immunologic reactions, thus making overwhelming infection the leading cause of death in transplant recipients.

Existing immunosuppresant treatment can differ in the case of different types of transplants. Liver allografts are less aggressively rejected than other organ allografts. For example, hyperacute rejection of a liver transplant does not occur invariably in patients who were presensitized to HLA antigens or ABO incompatibilities. Typical immunosuppressive therapy in an adult involves using cyclosporine, usually given IV at 4 to 6 mg/kg/day starting at the time of transplantation and then 8 to 14 mg/kg/day po when feeding is tolerated. Doses are adjusted downward if renal dysfunction occurs, and blood levels are used as approximate measures of adequate dosage.

In heart transplantation, immunosuppressive regimens are similar to those for kidney or liver transplantation. However, in lung and heart-lung transplants acute rejection occurs in > 80% of patients but may be successfully managed. Patients are treated with corticosteroids, given rapidly IV in high dosage, ATG, or OKT3. Prophylactic ALG or OKT3 is also frequently given during the first two posttransplant weeks. Pancreas transplantation is unique among the vascularized organ transplants: Instead of being used to save a life, it attempts to stabilize or prevent the devastating target organ complications of type I diabetes. Because the recipient exchanges the risks of insulin injection with the risks of immunosuppression, pancreas transplantation has been generally limited primarily to patients who already need to receive immunosuppressive drugs (i.e., diabetics with renal failure who are receiving a kidney transplant).

Patients with acute myeloid or lymphoblastic leukemia may benefit from bone marrow transplant (BMT). Pediatric BMT has expanded because of its potential for curing children with genetic diseases (e.g., thalassemia, sickle cell anemia, immunodeficiencies, inborn errors of metabolism). Another option for BMT is autologous transplantation (removal of a patient's own marrow when a complete remission has been induced, followed by ablative treatment of the patient with the hope of destruction of any residual tumor and rescue with the patient's own bone marrow). Since an autograft is used, no immunosuppression is necessary other than the short-term high-dose chemotherapy used for tumor eradication and bone marrow ablation; post-transplant problems with GVHD are minimal.

The rejection rate is < 5% in transplants for leukemia patients from HLA-identical donors. For multiply transfused patients with aplastic anemia, the rejection rate has also been significantly decreased because of increased immunosuppression during transplant induction. Nonetheless, complications can arise including rejection by the host of the marrow graft, acute GVHD, and infections. Later complications include chronic GVHD, prolonged immunodeficiency, and disease recurrence.

Graft versus Host Disease (GVHD) is more benefically treated by this invention than by administration of either a TNF-α antagonist or ECP alone. Chronic graft-versus-host disease (cGVHD) occurs in 30% to 60% of patients after allogeneic bone marrow transplantation (BMT). Both ECP and anti-TNFa therapy have shown positive effects in this disease but neither are complete and anti-TNFa has been associated with serious adverse events.

Numerous other transplantations can be made more effective with the combination treatment of the instant invention. Examples include, corneal transplantation, skin allografts, cartilage allografts, bone grafts, and small bowel transplants.

A host of other disorders can be treated more effectively using the instant invention. For example, cutaneous T-cell lymphoma is a disease in which T-lymphocytes become malignant and affect the skin. Three kinds of treatment are commonly used: radiation; chemotherapy; and photopheresis. Treatment of cutaneous T-cell lymphoma depends on the stage of the disease, and the patient's age and overall health. Standard treatment may be considered because of its effectiveness in patients in past studies, or participation in a clinical trial may be considered. Most patients with cutaneous T-cell lymphoma are not cured with standard therapy and some standard treatments may have more side effects than are desired. Treatment using the instant invention can be used in the treatment of this disease as well.

The present invention may also be used in implant surgery, for example, with implant surgery commonly performed in cosmetic or non- cosmetic plastic surgery. Such implants may include dental, fat grafting, for example to the cheeks, lips and buttocks, facial implants, including those to the nose, cheeks, forehead, chin and skull, buttocks implants, breast implants, etc. Other implants include, but are not limited to, corneal ring, cortical, orbital, cochlear, muscle (all muscles, including pectoral, gluteal, abdominal, gastrocnemius, soleus, bicep, tricep), alloplastic joint and bone replacement, bone repair implants (screws, rods, beams, bars, springs), metal plates, spinal, vertebral, hair, botox/collagen/restylane/perlane injections, penile implants, prostate seed implants, breast implants (cosmetic and reconstructive), interuterine devices, hormonal implants, fetal or stem cell implantation, pacemaker, defibrillator, artificial arteries/veins/valves, and artificial organs.

Autoimmune diseases can also be more effectively treated using the instant invention. These are diseases in which the immune system produces autoantibodies to an endogenous antigen, with consequent injury to tissues. Individuals may be identified as having a disease by several methods, including, but not limited to, HLA linkage typing, blood or serum-based assays, or identification of genetic variants, e.g., single nucleotide polymorphisms (SNPs). For example, once an individual is determined to have the HLA DR4 linkage and has been diagnosed to have rheumatoid arthritis, ECP and a TNF-α antagonist combination treatment can be prescribed. The TNF-α antagonist is inflimab. Other HLA alleles, also known as MHC alleles, that are associated with autoimmune diseases include B27 (Ankylosing spondylitis); DQA1*0501 and DQB1*0201 (Celiac disease); DRB1*03, DRB1*04, DQB1*0201, DQB1*0302, and DMA*0101 (Type I Diabetes); and Cw6 (Psoriasis). These alleles may also be used to determine whether an individual is experiencing an autoimmune disease and, thus, whether ECP and TNF-α antagonist combination treatment may be.

Blood or serum-based assays may be used to assess predisposition to a disease. There is, for example, an assay that detects the presence of autonuclear antibodies in serum, which may lead to the onset of lupus. Serum-based assays also exist for predicting autoimmune myocarditis. In addition, serum-based assays may be used to determine insulin levels (diabetes) or liver or heart enzymes for other diseases. T-3 levels may be predictive of Hashimotos thyroiditis. After an individual is determined to be having a disease using a blood or serum-based assay, the present invention may be used to prevent, or delay the onset of, or reduce the effects of these diseases. Individuals may be identified as being predisposed for disease through the identification of genetic variations, including, but not limited to, SNPs. Thus, in a further use of the invention, a determination is first made that a patient has an autoimmune disorder or is predisposed to one and that patient is then prescribed a combination of ECP (or other administration of apoptotic cells) and a TNF-α antagonist.

Standard diagnostic tests can be used to determine whether a patient has a disorder of the type described above.

### EXAMPLES

The following non-limiting examples further describe the invention.

In examples 1-5, Monocyte-derived dendritic cells were obtained as follows: PBMC were isolated from the peripheral blood of healthy donors by fractionation over Ficoll-Hypaque gradient centrifugation. Monocytes were positively selected using the MACS CD14 isolation kit and the Automacs system (Miltenyi Biotec, Germany). CD14⁺ monocytes were cultured in complete RPMI supplemented with 40 ng/ml IL-4 and GM-CSF (R&D Systems) for 5 days. Cytokine secretion was induced by stimulation of the dendritic cells with lypopolysacharides ("LPS", Sigma). Standard ELISA procedures were used to measure TNFα and IL-12 (R&D Systems) levels in culture supernatants.

### Example 1 (In Vitro Study of Inhibition of TNFα Production)

Freshly isolated CD14⁺ cells and monocyte-derived dendritic cells (5 X 10⁵ cells/well) were co-cultured in a 24-well tissue culture plate with ECP-treated CD15⁺ cells (2.5 X 10⁶ cells/well). After 2 hours, 0.5 mg/ml LPS was added to these cultures. After 24 hours of stimulation, supernants were collected from these cultures for cytokine measurements. ECP-treated cells were found to inhibit TNFα production from LPS-activated antigen-presenting cells.

### Example 2 (In Vitro Study of Inhibition of TNF α Production)

Monocyte-derived dendritic cells (1 X 10⁵ cells/well) were cultured in the presence of increasing quantities of LPS alone or with ECP-treated CD15⁺ cells (5 X 10⁵ cells/well), with 200 ng/ml infliximab mAb alone, or with the combination of the mAb and ECP-treated CD15⁺ cells. Culture supernatants were harvested from these cultures at 48 hours for measurement of TNFα production. Cells treated with infliximab mAb alone were found to have about 100 pg/ml TNFα. Those treated with ECP alone were found to have about 1000 pg/ml. While each of these treatments represent a reduction from the baseline value of over 1000 pg/ml, the levels dropped to an average of less than 50 pg/ml when the invention was used.

### Example 3 (In Vitro Study of Inhibition of TNFα Production)

Monocyte-derived dendritic cells (1 X 10⁵ cells/well) were cultured in the presence of 0.1 mg/ml LPS alone or with ECP-treated fresh CD15⁺ cells (5 X 10⁵ cells/well), with 200 ng/ml infliximab MAb alone, or with the combination of the mAb and ECP-treated CD15⁺ cells. Culture supernatants were harvested from these cultures at 48 hours for quantitation of TNFα production. Cells treated with infliximab mAb alone were found to have about 500 pg/ml TNFα. Those treated with ECP alone were found to have about 1700 pg/ml. While each of these treatments represent a reduction from the baseline value of over 2300 pg/ml, the levels dropped to about 100 pg/ml when the invention was used.

### Example 4 (In Vitro Study of Inhibition of TNFα Production)

Monocyte-derived dendritic cells (1 X 10⁵ cells/well) were cultured in the presence of increasing quantities of LPS alone or with ECP-treated CD15⁺ cells (5 X 10⁵ cells/well), with 200 ng/ml infliximab mAb alone, or with the combination of infliximab mAb and ECP-treated CD15⁺ cells. Culture supernatants were harvested from these cultures at 48 hours for measurement of TNFα production. Another group of dendritic cells were similarly treated and the culture supernatants were harvested from these cultures at 48 hours for quantitation of TNFα production. Cells treated with about 2ng/ml of infliximab mAb alone were found to have almost 1000 pg/ml TNFα. When this same dose of infliximab mAb was administered and ECP conducted the level dropped to about 1500 pg/ml. When 8 ng/ml of infliximab mAb were administered alone, the level was greater than 1300 pg/ml; the addition of ECP treatment lowered this to about 100 pg/ml. Doses of infliximab mAb of 40 ng/ml and greater with or without the combination of ECP reduced the level to less than 100 pg/ml. The effect of combined therapy was most pronounced at low levels of infliximab mAb administration (e.g., 2 ng/ml). Such doses are normally not considered therapeutic and demonstrate efficacy at levels at which adverse effects would not normally be expected.

### Example 5 (In Vitro Study of Inhibition of Other Pro-Inflammatory Cytokines)

Monocyte-derived dendritic cells (1 X 10⁵ cells/well) were cultured in the presence of increasing quantities of infliximab mAb either alone or with ECP-treated fresh CD15⁺ cells (5 X 10⁵ cells/well). Cells were then stimulated with 0.8 ng/ml LPS. Culture supernatants were harvested from these cultures at 48 hours for measurement of IL-12 production. IL-12 levels were reduced from a baseline value of about 150 pg/ml to about 125 pg/ml by the use of ECP. A combination of ECP and 2ng/ml infliximab mAb resulted in a reduction of IL-12 levels to about 10 pg/ml. When 200 ng/ml of infliximab mAb were employed in combination with ECP the IL-12 level was almost undetectable. Thus, the combination of ECP-treated cells and infliximab mAb significantly decreased IL-12 production by dendritic cells.

### Example 6 (Mouse Model In Vivo Application) (Prophetic)

### Mice

Male C3H/HeJ (C3H; H2k), (B6xC3H)F1 (H2bxk), (B6xDBA/2)F1 (H2bxd), C57BL/6 (B6; H2b), and CBA/JCr (CBA; H2k) mice will be purchased from the National Cancer Institute Research and Development Center (Frederick, MD). B10.BR (H2k) mice will be purchased from the Jackson Laboratories (Bar Harbour, ME). Mice used for experiments will be between 6-10 weeks of age, and housed in sterile microisolator cages within a specific pathogen-free facility, receiving autoclaved food and water ad libitum.

### Media

Phosphate-buffered saline (PBS) supplemented with 0.1% bovine serum albumin (BSA; Sigma Chemical Co., St Louis, MO) will be used for all in vitro manipulations of the donor bone marrow and lymphocytes. Immediately prior to injection, the cells will be washed and resuspended in PBS alone. For maintaining cell lines and for in vitro assays, RPMI 1640 medium (Mediatech, Herndon, VA) will be used, supplemented with 10% fetal bovine serum (FBS; GIBCO, Grand Island, NY), 2 mmol/L L-glutamine, 50 IU/mL penicillin, and 50 µg/mL streptomycin.

### Antibodies

The cV1q (aka. CNTO 2213) mAb, a rat/mouse Fc chimeric IgG2a construct with rat (Fab)₂ units specific for murine TNFα, and its isotype control M-T412, a human anti-CD4 mAb will be provided by Centocor, Malvern, PA. Ascites fluid containing mAb will be generated from hybridoma lines specific for either Thy-1.2 (J1j; ATTC TIB-184), CD4 (RL172), or CD8 (3.168) proteins, and will be used for the preparation of cellular grafts. Affinity-purified goat anti-mouse IgG (Cappel, Cosa Mesa, CA) will be used for B cell depletion. Guinea pig complement will be purchased from Rockland Immunochemicals (Gilbertsville, PA). Anti-CD3, anti-CD4, anti-CD8, anti-B220, and isotype control mAb, all coupled to phycoerythrin (PE), will be all purchased from BD Biosciences (Palo Alto, CA).

### Experimental Photopheresis

Splenocytes will be harvested from syngeneic littermate healthy mice and made into single cell suspension by grinding with the back end of a syringe in PBS. These cells will be re-suspended and cells washed twice with PBS before re-suspending at 12.5 x 10⁶ cells/mL PBS. Upon washing cells they will be resuspended in ice-cold medium and seeded at approximately 10⁶ cells/ml in a T75 flask. Psoralen (UVADEX solution) will be added to a final concentration of 200 ng/ml, which is a 100 fold dilution from the stock solution provided by Therakos. The flask will be placed lying down in the UVA irradiation chamber and given approximately 1.5 J/cm2 of light which corresponds to 1.5 minutes of bottom light when the tray is 6 cm from the light source. Cells will be quickly removed from the flask to avoid adherence and placed at the appropriate concentration for injection. If there is adherence, the flask will be gently scraped or tapped to remove most of the cells.

### Bone Marrow Transplantation

Bone marrow will be harvested from the tibia and femurs of donor mice by flushing with PBS containing 0.01% BSA (PBS/BSA). Bone marrow cells will be depleted of T cells using an anti-Thy 1.2 mAb (J1j; American Type Culture Collection, Rockville, MD) at a 1:100 dilution and guinea pig complement (Rockland Immunochemicals, Gilbertsville, PA) at a dilution of 1:6 for 45 minutes at 37°C. Lymphocytes will be isolated from spleens and lymph nodes of donor mice. Splenocytes will be treated with Gey's balanced salt lysing solution containing 0.7% ammonium chloride (NH₄Cl) to remove red blood cells (RBCs). After RBC depletion, spleen and lymph node cells will be pooled and depleted of B cells by panning on a plastic petri dish, precoated with a 5 mg/ml dilution of goat anti-mouse IgG for 1 hour at 4°C. These treatments are expected to result in donor populations of approximately 90%-95% CD3⁺ cells, as quantitated by fluorescent flow cytometry. T cells subsets will be then isolated via negative selection using either anti-CD8 (3.168) or anti-CD4 mAb (RL172) and complement. These treatments are expected to reduce the targeted T cell subset populations to background levels, as determined by flow cytometric analysis. Recipient mice will be exposed to 13 Gy whole body irradiation from a ¹³⁷CS source at 1.43 Gy/min, delivered in a split dose of 6.5 Gy each, separated by 3 hours. These mice will be then transplanted with 2x10⁶ anti-Thy 1.2 treated bone marrow cells (ATBM; T cell-depleted) along with the indicated number of appropriate T cells (donor CD4 or CD8 enriched T cells), intravenously (i.v.) via the tail vein. Mice will be treated with cV1q anti-TNF-a or isotype control M-T412 mAb (1 mg; i.p.) 1 day before transplantation and again on days 0, 4, 8, and 12 (all at 0.5 mg; i.p.). For GVL experiments, B6 recipient mice will be challenged with an injection of MMB3.19 cells (1x10⁵ in 0.5 mL PBS; i.p.) one day before transplantation of donor ATBM and T cells, with a similar schedule of anti-TNFα mAb treatment. In both GVHD and GVL experiments, the mice will be checked daily for morbidity and mortality until completion. The data will be pooled from 2-3 separate experiments, and median survival times (MST) will be determined as the interpolated 50% survival point of a linear regression through all of the day of death data points, including zero. Statistical comparisons for survival between experimental groups will be performed by the nonparametric Wilcoxon signed rank test. Significance for weight comparisons will be determined by the T-test at individual time points.

### Flow cytometry

Appropriate mAbs in volumes of 25 µL will be incubated with 2-5x10⁵ cells in the wells of a 96-well U-bottom microplate at 4°C for 30 minutes, centrifuged at 1500 rpm for 3 minutes, and washed with PBS containing 0.1% BSA and 0.01% sodium azide (wash buffer). The percentage positive cells and the arithmetic mean fluorescence intensity will be calculated for each sample.

### Pathological Analysis

Full thickness ear biopsies (3x2 mm) will be sampled from each mouse of the various treatment groups and immediately fixed in 4% glutaraldehyde overnight and then rinsed with 0.1M sodium cacodylate buffer (pH 7.4). Tissues will be post-fixed with 2% osmium tetroxide for 2 h, dehydrated in graded ethanol and embedded in Epon 812. One-micron-thick sections will be cut with a Porter-Blum MT2B ultramicrotome, stained with toluidine blue, and finally dipped in 95% ethanol for light microscopic analysis. The number of dyskeratotic epidermal cells/linear mm, as previously determined, will be counted under a x100 objective and a x10 eye piece of a light microscope. More than ten linear mm of the epidermis will be assessed in each animal and each time point. The analysis will be performed under blinded conditions as to the treatment groups.

### Effect of anti- TNFα mAb on CD8 T cell-mediated GVHD

To determine if anti- TNFα mAb treatment could affect the development of CD8⁺ T cell-mediated GVHD, the MHC-matched, miHA-disparate B10.BRàCBA GVHD model will be utilized, as it has a well-established etiology. CBA mice will be lethally irradiated (13 Gy, split dose) and transplanted with B10.BR ATBM cells (2x10⁶), alone, or in addition to a highly enriched population (95%) of CD8⁺ T cells (3x106). Mice will be either left untreated, treated with the isotype-matched control MT412 mAb, or the anti-TNFα mAb (cV1q) mAb on day -1 (1 mg, i.p.) and days 0, 4, 8, & 12 of transplant (0.5 mg; i.p.). Whereas all recipients of ATBM cells alone will survive for at least 70 days, mice transplanted with donor T cells, and left untreated or treated with control MT412 mAb, will succumb to GVHD with similar MST values of approximately 20 days. In contrast, CBA recipients of donor T cells, but administered cV1q mAb, will exhibit approximately 40% survival with a MST of approximately 50 days which will be significantly different than the MT412 control group. In addition, surviving anti-TNFα mAb treated mice will not display evident symptoms of GVHD (e.g., ruffled fur, skin lesions, hunched posture, or diarrhea), and their body weights will be at a relatively constant level ranging 5-12% below that of the control ATBM transplanted group. The mice that do develop fatal GVHD in the presence of cV lq will do so with slower kinetics than the untreated or MT412-treated groups. When cV1q is administered at 0.1mg i.p. there will be no significant decrease in GvHD onset.

Experimental ECP will be administered by i.v. injection of 10⁷ syngeneic splenocytes from a littermate control mouse on the same day as the BMT and 3 days later. CBA recipients of donor T cells, but administered ECP-treated cells, will exhibit approximately 20% survival with a MST of approximately 30 days which will be significantly different than the control group. In addition, surviving ECP-treated mice will display decreased evidence of GVHD symptoms (e.g., ruffled fur, skin lesions, hunched posture, or diarrhea), and their body weights will be at a relatively constant level ranging 5-20% below that of the control ATBM transplanted group. The mice that do develop fatal GVHD in the presence of ECP-treated cells will do so with slower kinetics than the untreated groups.

The combination of ECP treatment with sub-efficacious doses of anti-TNFα treatment will be superior to either treatment alone. CBA recipients of donor T cells, but administered cV1q mAb at 0.1mg along with ECP, will exhibit approximately 60% survival with a MST of approximately 70 days which will be significantly different than the control groups. In addition, surviving dual treated mice will not display evident symptoms of GVHD (e.g., ruffled fur, skin lesions, hunched posture, or diarrhea), and their body weights will be at a relatively constant level ranging 5-12% below that of the control ATBM transplanted group. The mice that do develop fatal GVHD in the presence of dual therapy will do so with slower kinetics than the untreated groups and slower than the ECP or anti TNF groups alone.

### Effect of anti-TNFα mAb on GVHD across an MHC barrier

The haploidentical C3Hà(B6xC3H)F1 mouse model will be utilized to determine if the neutralization of TNFα by cV1q treatment could affect the course of GVHD across a full MHC barrier. C3H T cells (both CD4+ and CD8+; 5x10⁶) and ATBM cells (2x10⁶) will be transplanted i.v. into lethally irradiated (13 Gy, split dose) (B6xC3H)F1 mice, which induces a rapid acute GVHD response characterized by severe weight loss and early fatality (MST of approximately 5 days). Similar results will be obtained in recipients treated with control MT412 mAb, but those mice treated with cV1q (1 mg i.p. on day -1 and 0.5 mg on days 0, 4, 8, &12) will exhibit approximately 40% long-term survival with a MST of about 40 days which will be significantly different compared to either untreated or the MT412 control groups. Treatment with 0.1 mg of cv1q will have a non-significant but notable effect on GvHD onset.

Experimental ECP will be administered by i.v. injection of 10⁷ syngeneic splenocytes from a littermate control mouse on the same day as the BMT and 3 days later. CBA recipients of donor T cells, but administered ECP-treated cells, will exhibit approximately 20% survival with a MST of approximately 10 days which will be different but not significantly different than the control group. In addition, surviving ECP-treated mice will display decreased evidence of GVHD symptoms (e.g., ruffled fur, skin lesions, hunched posture, or diarrhea), and their body weights will be at a relatively constant level ranging 5-20% below that of the control ATBM transplanted group. The mice that do develop fatal GVHD in the presence of ECP-treated cells will do so with slower kinetics than the untreated groups.

The combination of ECP treatment with sub-efficacious doses of anti-TNFα treatment will be superior to either treatment alone. CBA recipients of donor T cells, but administered cV1q mAb at 0.1mg along with ECP, will exhibit approximately 60% survival with a MST of approximately 70 days which will be significantly different than the control groups. In addition, surviving dual treated mice will not display evident symptoms of GVHD (e.g., ruffled fur, skin lesions, hunched posture, or diarrhea), and their body weights will be at a relatively constant level ranging 5-12% below that of the control ATBM transplanted group. The mice that do develop fatal GVHD in the presence of dual therapy will do so with slower kinetics than the untreated groups and slower than the ECP or anti TNF groups alone.

In terms of weight loss, after an initial slight drop in the first few days due to the irradiation conditioning, the control ATBM mice will steadily gain weight throughout the remainder of the experiment. On the other hand, the untreated and MT412-treated groups transplanted with donor T cells will never recover from the initial drop and will likely instead continue to rapidly lose weight until their death, consistent with severe GVHD. However, the cV1q anti-TNFα mAb-treated mice will recover somewhat by day 9 and surviving animals after day 37 will continue to gain weight during the remaining course of the experiment, tracking approximately 6-12% below the ATBM group. Animals treated with 0.1 mg of cvlq will lose weight at only a slightly better kinetics, albeit insignificantly different, than control animals. ECP treated animals will have a significantly improved weight gain and the combination of anti TNF and ECP will be virtually identical to control animals not given a BMT or the 1mg anti TNF groups.

### Effect of anti- TNFα mAb on CD4+ T cell-mediated GVHD

Since donor CD4⁺ T cell responses tend to dominate the development of GVHD in the C3Hà(B6xC3H)F1 model and in light of the initial observation of a moderate effect of anti- TNFα mAb treatment when a complete donor T cell inoculum was transplanted, we will focus our attention on the CD4-mediated GVHD component. The injection of 3x10⁶ C3H CD4⁺ T cells together with 2x10⁶ ATBM cells into irradiated (13 Gy, split dose) (B6xC3H)F1 mice will result in the majority of the untreated (about 75%; MST of 10-30 days) and control MT412-treated (about 80%; MST of 10-30 days) mice succumbing to severe acute GVHD. In contrast, 100% of the mice treated with the cV1q anti- TNFα mAb (1 mg i.p. on day -1 and 0.5 mg on days 0, 4, 8, &12) will survive beyond 60 days. These mice will not exhibit any visible symptoms of GVHD and rapidly recover from their initial body weight loss following irradiation and continue to gain weight until the end of the experiment in parallel to the ATBM control group. The highly significant effect of cV1q treatment on survival in the CD4-mediated GVHD will suggest that the more modest effect observed previously with transfer of a whole donor T cell inoculum will be likely due to less inhibition of CD8-mediated anti-MHC class I responses. However, this can not be tested directly in this model, because purified C3H CD8⁺ T cells are unable to mediate lethal GVHD on their own, without the presence of CD4⁺ T cells.

Treatment with 0.1 mg of cV1q anti TNFα antibodies will have a more modest effect at inhibiting GvHD. Approximately 40% of animals will survive past 60 days. The surviving mice will show initial signs of GvHD but they will fade and weight loss will not improve as fast as in the 1mg cV1q group but will be significantly different than controls.

Experimental ECP will be administered by i.v. injection of 10⁷ syngeneic splenocytes from a littermate control mouse on the same day as the BMT and 3 days later. F1 recipients of donor T cells, but administered ECP-treated cells, will exhibit approximately 20% survival with a MST of approximately 10-25 days which will be different but not significantly different than the control group. In addition, surviving ECP-treated mice will display decreased evidence of GVHD symptoms (e.g., ruffled fur, skin lesions, hunched posture, or diarrhea), and their body weights will be at a relatively constant level ranging 5-20% below that of the control ATBM transplanted group. The mice that do develop fatal GVHD in the presence of ECP-treated cells will do so with slower kinetics than the untreated groups.

The combination of ECP treatment with sub-efficacious doses of anti-TNFα treatment will be superior to either treatment alone. CBA recipients of donor T cells, but administered cV1q mAb at 0.1mg along with ECP, will exhibit approximately 90% survival at day 60 which will be significantly different than the control groups. In addition, surviving dual treated mice will not display evident symptoms of GVHD (e.g., ruffled fur, skin lesions, hunched posture, or diarrhea), and their body weights will be at a relatively constant level ranging 5-12% below that of the control ATBM transplanted group. The mice that do develop fatal GVHD in the presence of dual therapy will do so with slower kinetics than the untreated groups and, although not statistically significant, slower than the ECP or anti TNF groups alone.

### Example 7 (Human Application to synergize and lower toxicity of anti-TNF-α therapy alone) (Prophetic)

### Example summary

This example will demonstrate that the intensity regimen of anti-TNFα along with ECP has a significantly better toxicity profile than those proposed in the literature. Initially 1mg/kg of infliximab anti TNF α will be used. However, the range of useful doses may range from 0.1 mg/kg to 3 mg/kg.

### Patients

Patients will receive an allogeneic hematopoietic stem cell (HSC) transplant using standard regimen's dictated by the sites and the protocol agreed upon and will not be limited to the following medications; included oral and intravenous corticosteroids, cyclosporine, tacrolimus, sirolimus, mycophenolate mofetil (MMF). Patients receiving nonmyeloablative HSC transplants will receive conditioning chemotherapy with busulfan and fludarabine, and overall different GVHD prophylaxis regimens. CMV serostatus of donor-recipient pairs and median follow-up times will be similar. ECP will be administered using standard procedure prior to HSC and possibly at various times following HSC. Infliximab will be administered following HSC at a dose of 0.5 mg/kg. Patient's will be followed and scored using standard procedures such as the modified Glucksberg scale and data will be collected on GVHD prophylaxis regimen, date of onset, and maximum overall and organ-specific grade.

IFIs will be classified according to the 2002 European Organisation for Research and Treatment of Cancer (EORTC)/National Institute of Allergy and Infectious Deseases (NIAID) international consensus. Cases of IFI will be identified by review of the medical records of all patients identified in the cohort and by review of all pathology, microbiology, infection control, and radiology databases. Physicians will document their findings without knowledge of infliximab exposure. Only proven or probable IFI not due to *Candida* species will be considered for the analysis. IFI date will be documented as the day when the diagnostic procedure will be performed for proven IFI, or the day when both radiology and microbiology data will be available to the clinician for probable IFI. If a diagnosis of IFI will be made after death, the IFI date will be considered the date of death, but if a probable IFI diagnosis will be confirmed at postmortem examination, the IFI date will be documented as the day when the probable IFI diagnosis will be made.

All doses of any corticosteroid received by patients with severe GVHD will be transformed into prednisone equivalents using the corticosteroid equivalence table. The cumulative corticosteroid dose, adjusted to body weight, will be calculated from the day of HSCT until death, the development of IFI, the end of cohort follow-up period, or when corticosteroids where tapered below 20 mg/d for more than 30 days. Empiric and prophylactic antifungal use will be documented.

Surviving patients will be censored on that date or on the last visit before that date. The study will be approved by appropriate administrative/regulatory bodies.

### Statistical analysis

The 2-sided Fisher exact test, Wilcoxon test, or *t* test will be used as appropriate for comparison of baseline characteristics. IFI incidence rates and incidence rate ratios will be calculated according to different exposure categories from day of transplantation in the HSCT cohort, and from day of onset of GVHD in those who developed severe GVHD; patients will be censored at death or last visit before the end of follow-up. Confidence intervals for incidence rates and incidence rate ratios will be calculated using the Haensze and Byar method, respectively. Kaplan-Meier curves will be calculated for survival and for time to IFI from date of transplantation. In those patients with severe GVHD, time to IFI from onset of acute GVHD will be also calculated. Times to event will be compared by using the log-rank test. Time-dependent Cox regression analysis of time to IFI from onset of GVHD will be done to control for possible confounding or interactions among variables for patients with severe GVHD. Univariate Cox models will be calculated for all possible risk factors among patients with severe GVHD. All covariates with a P value of less than .2 on univariate Cox analysis of IFI will be considered in the multivariable Cox model. Infliximab will be modeled as a time-dependent variable; its exposure will be assumed constant once weekly infusions will be initiated. Only candidate variables that will be statistically significantly associated (P < .05) with IFI in the final model will be retained unless significant confounding will be noted. The SAS System for Windows, version 8.01 (SAS Institute, Carey, NC), will be used for the above analyses.

### Results

### Incidence of and treatments for acute GVHD

ECP will be administered approximately 2 times prior to the HSC at days -10 to -4 prior to HSC. In addition, the ECP therapy will be administered approximately weekly to further prevent development of acute GvHD during the first 100 days following transplant. A preliminary analysis will demonstrate similar survival and IFI rates among patients with no GVHD and those with grades I to II GVHD; consequently, these groups will be pooled together into no or non-severe GVHD. Severe GVHD will be defined as an overall grade of III or IV. Approximately 20% in the cohort will develop acute severe GVHD. The proportion of unrelated donors will be higher in patients with severe GVHD when compared with the rest of the cohort; otherwise, the baseline characteristics will be similar. Among myeloablative and nonmyeloablative HSC transplant recipients, the proportion of any degree of GVHD or severe GVHD will be similar.

Patients diagnosed with severe GVHD may receive multiple medications that may include MMF and corticosteroids at an initial dose of at least 2 mg/kg/d, tapered to response, and the addition or increase in dose of a calcineurin inhibitor or sirolimus.

Infliximab administration will be initiated approximately 10-50 days after the initial diagnosis of acute GVHD. Patients will receive 2-15 doses of 1 mg/kg on a weekly or biweekly basis. When compared with patients who did not receive infliximab, recipients will be more likely to have signs and symptoms of GVHD.

### IFIs in the cohort

Proven or probable IFIs not due to *Candida* species (aspergillosis, zygomycosis, etc.) will be diagnosed in the cohort during the observation period.

The overall IFI IR among patients with severe GVHD will be approximately 1 case/1000 GVHD patient-days. Among baseline characteristics, non-myeloablative HSCT will be associated with a significantly increased IFI IR of approximately 3 cases/1000 GVHD patient-days, whereas myeloablative HSC transplant recipients who developed severe GVHD will have an IFI IR of less than 1 case/1000 GVHD patient-days. Characteristics of non-myeloablative HSCT protocols, such as conditioning regimen, receiving peripheral blood stem cells, and cyclosporine use for GVHD prophylaxis, will be also associated with a slightly higher risk of IFI.

The time to IFI from the onset of GVHD among patients with severe GVHD will be stratified by 3 mg/kg infliximab use. There will be a significantly higher probability of IFI in the infliximab recipients. Treatment with ECP will not show a statistically significant increase in IFI.

A time-dependent Cox regression analysis model for developing IFI in patients with severe GVHD will be developed. Univariate hazard ratios (HRs) will be calculated for all possible IFI risk factors Only characteristics with an unadjusted HR P values of less than .20 will be considered in the multivariate model.

Variables that will be collinear with a nonmyeloablative HSCT described will be not included separately, and given that 10 IFIs will be being analyzed, 2 covariates with the highest HR and P values of less than .05 will be retained in the final model. Given that infliximab will be given preferentially to patients with severe gastrointestinal GVHD and that gastrointestinal organ-specific grade 3 or 4 will be found to be significantly associated with IFI on univariate Cox, this covariate will be kept in the final model to minimize confounding by indication, even though it became nonsignificant in the presence of other covariates modeled. The adjusted HR of infliximab use, as a time-dependent covariate, will be approximately 14 ; the adjusted HR of nonmyeloablative HSCT will be approximately 8. The adjusted HR of severe gastrointestinal organ-specific grade 3 or 4 GVHD will be approximately 4 in the presence of infliximab use and transplant type as covariates. The time to IFI hazard function plots of 3 mg/kg infliximab exposure will show increasing hazard over time. Use of 1mg/kg or less of infliximab will not reveal a significant increase in HR. ECP alone will show an HR not significantly different from those patients treated with standard regimen only. The combination of low dose infliximab with ECP will have a significantly lower HR than high dose infliximab alone. Taken together with the increased efficacy this treatment regimen is a more effective, safer treatment modality.

### Survival

The median survival of the whole cohort at the end of follow-up will be approximately 250-400 days. When stratified according to GVHD severity, the median survival of patients with severe GVHD will be significantly lower than that of patients with no or non-severe GVHD. Among patients with severe GVHD, the median survival of 3mg/kg infliximab recipients may be significantly lower than that of non-recipients. ECP treated patients will have a significant survival pattern over standard regimen patients or patients receiving low dose infliximab alone. Lowering the dose of infliximab to 1mg/kg along with the standard ECP regimen will lead to significant improvements in GvHD score yet the IFI associated with higher levels of infliximab will be dramatically and statistically reduced.

## Claims

1. A combined product comprising:
a) an *ex vivo* population of cells that has been subjected to an apoptosis-inducing treatment *ex vivo;* and
b) infliximab in a dose that does not exceed 3mg/kg,
for combined, simultaneous or sequential administration to a patient for use in treating an autoimmune disease or ameliorating one or more symptoms thereof.

2. The product for use according to claim 1, for simultaneous administration.

3. The product for use according to claim 1 or claim 2, wherein the population of cells is obtained from a portion of blood.

4. The product for use according to claim 3, wherein, the cells are autologous leukocytes.

5. The product for use according to any one of claims 1 to 4, wherein the population of cells has been subjected to an apoptosis-inducing treatment which is an extracoporeal photopheresis (ECP) procedure that employs a photoactivatable compound together with light of a wavelength that activates said photoactivatable compound.

6. The product for use according to claim 5, wherein the photoactivatable compound is a psoralen and the light is UVA.

7. The product for use according to claim 6, wherein the psoralen is 8-methoxypsoralen (8-MOP).

8. The product for use according to any one of claims 1 to 7, for use in treating systemic lupus erythematosus (SLE) rheumatoid arthritis, thyroidosis, graft versus host disease, scleroderma, diabetes mellitus, Graves' disease; sarcoidosis, chronic inflammatory bowel disease, ulcerative colitis and Crohn's pathology.

9. The product for use according to any one of claims 1 to 7, for use in treating graft versus host disease.

10. The product for use according to any one of claims 1 to 7, for use in administration to a transplant recipient.

11. The product for use according to claim 10, wherein components a) and b) are adapted to be administered according to a schedule selected from the group consisting of two days, one week prior to the transplantation; three days, one week prior to harvesting said transplant; two days a week for two weeks prior to the transplantation; and three days a week for three weeks prior to the transplantation.

12. The product for use according to any one of claims 1 to 7, wherein said population of cells is an autologous population obtained from the patient before a transplant and wherein the product further comprises a second population of autologous cells obtained from the patient after said patient has received said transplant, wherein said second population of cells has been subjected to the apoptosis-inducing treatment.

13. The product for use according to claim 12, wherein components a) and b) are adapted to be administered according to a schedule selected from the group consisting of two days, one week prior to said recipient receiving said implant; three days, one week prior to said recipient receiving said implant; two days a week for two weeks prior to said recipient receiving said implant; and three days a week for three weeks prior to said recipient receiving said implant; and step c) is carried out according to a schedule selected from the group consisting of weekly, monthly, twice a month, three times a month, every other month, every three months, every six months, and yearly.

14. A composition comprising infliximab for use in a method of treating a patient with an autoimmune disorder or the predisposition for an autoimmune disorder wherein said method comprises testing the patient to determine whether the patient has an autoimmune disorder, and administering infliximab in a dose that does not exceed 3mg/kg and ECP if such patient has an autoimmune disorder or a predisposition to such disorder.

## Patentansprüche

1. Kombiniertes Produkt, umfassend:
a) eine *ex*-*vivo*-Population von Zellen, die *ex vivo* einer Apoptose-induzierenden Behandlung unterworfen worden ist; und
b) Infliximab in einer Dosis, die 3 mg/kg nicht übersteigt,
für kombinierte, gleichzeitige oder sequentielle Verabreichung an einen Patienten zur Verwendung bei der Behandlung einer Autoimmunerkrankung oder Linderung von einem oder mehreren Symptomen davon.

2. Produkt zur Verwendung nach Anspruch 1, zur gleichzeitigen Verabreichung.

3. Produkt zur Verwendung nach Anspruch 1 oder Anspruch 2, wobei die Population von Zellen aus einer Portion Blut erhalten ist.

4. Produkt zur Verwendung nach Anspruch 3, wobei die Zellen autologe Leukozyten sind.

5. Produkt zur Verwendung nach einem der Ansprüche 1 bis 4, wobei die Population von Zellen einer Apoptose-induzierenden Behandlung unterworfen worden ist, die ein extrakorporales Photopherese(ECP)-Verfahren ist, das eine photoaktivierbare Verbindung einsetzt, zusammen mit Licht einer Wellenlänge, die die photoaktivierbare Verbindung aktiviert.

6. Produkt zur Verwendung nach Anspruch 5, wobei die photoaktivierbare Verbindung ein Psoralen ist und das Licht UVA ist.

7. Produkt zur Verwendung nach Anspruch 6, wobei das Psoralen 8-Methoxypsoralen (8-MOP) ist.

8. Produkt zur Verwendung nach einem der Ansprüche 1 bis 7, zur Verwendung bei der Behandlung von systemischem Lupus erythematosus (SLE), rheumatoider Arthritis, Thyroidose, Graft-versus-host-Erkrankung, Sklerodermie, Diabetes mellitus, Graves-Erkrankung, Sarkoidose, chronischer entzündlicher Darmerkrankung, Colitis ulcerosa und Crohn-Pathologie.

9. Produkt zur Verwendung nach einem der Ansprüche 1 bis 7, zur Verwendung bei der Behandlung von Graft-versus-host-Erkrankung.

10. Produkt zur Verwendung nach einem der Ansprüche 1 bis 7, zur Verwendung bei der Verabreichung an einen Transplantat-Empfänger.

11. Produkt zur Verwendung nach Anspruch 10, wobei Komponenten a) und b) so angepasst sind, dass sie entsprechend einem Schema verabreicht werden, das ausgewählt ist aus der Gruppe, bestehend aus zwei Tagen, eine Woche vor der Transplantation; drei Tagen, eine Woche vor der Entnahme des Transplantats; zwei Tagen pro Woche für zwei Wochen vor der Transplantation; und drei Tagen pro Woche für drei Wochen vor der Transplantation.

12. Produkt zur Verwendung nach einem der Ansprüche 1 bis 7, wobei die Population von Zellen eine autologe Population ist, die vom Patienten vor einem Transplantat erhalten worden ist, und wobei das Produkt weiter eine zweite Population von autologen Zellen umfasst, die vom Patienten erhalten worden ist, nachdem der Patient das Transplantat erhalten hat, wobei die zweite Population von Zellen der Apoptose-induzierenden Behandlung unterworfen worden ist.

13. Produkt zur Verwendung nach Anspruch 12, wobei Komponenten a) und b) so angepasst sind, dass sie entsprechend einem Schema verabreicht werden, das ausgewählt ist aus der Gruppe, bestehend aus zwei Tagen, eine Woche bevor der Empfänger das Implantat erhält; drei Tagen, eine Woche bevor der Empfänger das Implantat erhält; zwei Tagen pro Woche für zwei Wochen, bevor der Empfänger das Implantat erhält; und drei Tagen pro Woche für drei Wochen, bevor der Empfänger das Implantat erhält; und Schritt c) gemäß einem Schema durchgeführt wird, das ausgewählt ist aus der Gruppe, bestehend aus wöchentlich, monatlich, zweimal monatlich, dreimal monatlich, jeden zweiten Monat, jeden dritten Monat, jeden sechsten Monat und jährlich.

14. Zusammensetzung, umfassend Infliximab, zur Verwendung in einem Verfahren zur Behandlung eines Patienten mit einer Autoimmunerkrankung oder der Prädisposition für eine Autoimmunerkrankung, wobei das Verfahren das Testen des Patienten umfasst, um zu bestimmen, ob der Patient eine Autoimmunerkrankung hat, und Verabreichen von Infliximab in einer Dosis, die 3 mg/kg nicht übersteigt, und ECP, wenn ein solcher Patient eine Autoimmunerkrankung oder eine Prädisposition für eine solche Erkrankung hat.

## Revendications

1. Produit combiné comprenant:
a) une population *ex vivo* de cellules qui a été soumise à un traitement d'induction d'apoptose *ex vivo;* et
b) infliximab en une dose qui ne dépasse pas 3 mg/kg,
pour une administration combinée, simultanée ou séquentielle à un patient pour utilisation dans le traitement d'une maladie autoimmune ou pour améliorer un ou plusieurs symptômes de celle-ci.

2. Produit pour utilisation selon la revendication 1, pour une administration simultanée.

3. Produit pour utilisation selon la revendication 1 ou la revendication 2, où la population de cellules est obtenue à partir d'une portion de sang.

4. Produit pour utilisation selon la revendication 3, où les cellules sont des leucocytes autologues.

5. Produit pour utilisation selon l'une quelconque des revendications 1 à 4, où la population de cellules a été soumise à un traitement d'induction d'apoptose qui est une procédure de photophorèse extracorporelle (ECP) qui utilise un composé photoactivable conjointement avec la lumière d'une longueur d'onde qui active ledit composé photoactivable.

6. Produit pour utilisation selon la revendication 5, où le composé photoactivable est un psoralène, et la lumière est UVA.

7. Produit pour utilisation selon la revendication 6, où le psoralène est 8-méthoxypsoralen (8-MOP).

8. Produit pour utilisation selon l'une quelconque des revendications 1 à 7, pour utilisation dans le traitement du lupus érythémateux disséminé (SLE), de la polyarthrite rhumatoïde, thyroïdosis, réaction de greffe contre hôte, sclérodermie, diabète sucré, maladie de Grave; sarcoïdose, maladie chronique inflammatoire de l'intestin, recto-colite hémorragique et la pathologie de Crohn.

9. Produit pour utilisation selon l'une quelconque des revendications 1 à 7, pour utilisation dans le traitement de la réaction de greffe contre hôte.

10. Produit pour utilisation selon l'une quelconque des revendications 1 à 7, pour utilisation dans l'administration à un receveur de greffe.

11. Produit pour utilisation selon la revendication 10, où les composants a) et b) sont aptes à être administrés en accord avec un programme sélectionné dans le groupe consistant en deux jours, une semaine avant la greffe; trois jours, une semaine avant la récolte de ladite greffe; deux jours, une semaine à deux semaines avant la transplantation; et trois jours une semaine à trois semaines avant la transplantation.

12. Produit pour utilisation selon l'une quelconque des revendications 1 à 7, où ladite population de cellules est une population autologue obtenue du patient avant la greffe, et où le produit comprend en outre une seconde population de cellules autologues obtenue du patient après que ledit patient a reçu ladite greffe, où ladite seconde population de cellules a été soumise à un traitement d'induction d'apoptose.

13. Produit pour utilisation selon la revendication 12, où les composants a) et b) sont aptes à être administrés selon un programme sélectionné dans le groupe consistant en deux jours, une semaine avant que ledit récipient reçoit ledit implant; trois jours, une semaine avant que ledit récipient reçoit ledit implant; deux jours une semaine à deux semaines avant que ledit receveur reçoit ledit implant; et trois jours une semaine à trois semaines avant que ledit receveur reçoit ledit implant; et l'étape c) est exécutée en accord avec un programme sélectionné dans le groupe consistant en hebdomadaire, mensuel, deux fois par mois, trois fois par mois, un mois sur deux, un mois sur trois, un mois sur six et annuel.

14. Composition comprenant infliximab pour utilisation dans une méthode de traitement d'un patient atteint d'un trouble autoimmun ou prédisposé à un trouble autoimmun, où ladite méthode comprend le test du patient pour déterminer si le patient présente un trouble autoimmun, et l'administration de infliximab en une dose qui ne dépasse pas 3mg/kg et ECP si un tel patient a un trouble autoimmun ou est prédisposé à un tel trouble.
